# EUROPÄISCHE PATENTSCHRIFT

(11) **EP 1 527 340 B1**
(45) Veröffentlichungstag und Bekanntmachung des Hinweises auf die Patenterteilung: **28.10.2009**
(21) Anmeldenummer: 03792196.2
(22) Anmeldetag: 14.07.2003
(51) Int. Cl.: G01N 33/50

(54) **VERFAHREN ZUR IDENTIFIZIERUNG VON SUBSTANZEN**
METHOD FOR IDENTIFYING SUBSTANCES
METHODE D'IDENTIFICATION DE SUBSTANCES

(30) Priorität: 23.07.2002 DE 10233516
(43) Veröffentlichungstag der Anmeldung: 04.05.2005
(73) Patentinhaber: Sanofi-Aventis Deutschland GmbH, 65929 Frankfurt am Main (DE)
(72) Erfinder: GRATZER, Sabine, 82166 Gräfelfing (DE); DE HOOP, Meltsje, 65719 Hofheim (DE); MAI, Bernhard, 82223 Eichenau (DE)
(86) Internationale Anmeldenummer: PCT/EP2003/007569
(87) Internationale Veröffentlichungsnummer: WO 2004/019036

(56) Entgegenhaltungen:
- EP-A- 0 174 477
- EP-A- 0 974 649
- WO-A-00/12704
- US-A- 5 691 188
- US-A- 5 728 534
- US-A- 5 885 769
- ARNOLD S F ET AL: "A YEAST ESTROGEN SCREEN FOR EXAMINING THE RELATIVE EXPOSURE OF CELLS TO NATURAL AND XENOESTROGENS" ENVIRONMENTAL HEALTH PERSPECTIVES, XX, XX, Bd. 104, Nr. 5, Mai 1996 (1996-05), Seiten 544-548, XP001026841 ISSN: 0091-6765
- PAUSCH M H: "G-protein-coupled receptors in Saccharomyces cerevisiae: high-throughput screening assays for drug discovery" TRENDS IN BIOTECHNOLOGY, ELSEVIER PUBLICATIONS, CAMBRIDGE, GB, Bd. 15, Nr. 12, 1. Dezember 1997 (1997-12-01), Seiten 487-494, XP004097426 ISSN: 0167-7799

## Beschreibung

Die vorliegende Erfindung betrifft ein zelluläres Verfahren zur Identifizierung von Substanzen, die fähig sind, die Aktivität eines Zielmoleküls zu beeinflussen. Bei der Entwicklung neuer Medikamente, die auf zelluläre Zielmoleküle einwirken, werden herkömmlicherweise biochemische oder zelluläre funktionelle Assays eingesetzt, durch die eine Vielzahl putativer Wirksubstanzen daraufhin untersucht werden kann, ob sie eine Wirkung auf das zu untersuchende Zielmolekül ausüben. Zelluläre Assays arbeiten herkömmlicherweise auf der Grundlage wachstumsbasierender Testsysteme, bei denen sich die Aktivität des zu untersuchenden Zielmoleküls auf die Zellvermehrung auswirkt. Überdies sind Testsysteme bekannt, bei denen die Aktivität des Zielmoleküls anhand der Aktivität bzw. Expression sogenannter Reportergenprodukte detektierbar und quantifizierbar ist. Beide Arten von Assays erlauben es zwar, auch im Hochdurchsatz (High throughput, HTS) Screen neue Wirksubstanzen zu identifizieren, die die Aktivität des Zielmoleküls modulieren; der Nachteil dieser bekannten Testsysteme liegt jedoch darin, dass ihr Signal-zu-Hintergrund Verhältnis in der Regel relativ klein ist, so dass ihre Spezifität, insbesondere bei Einsatz im HTS Format sehr gering ist. Angesichts der genannten Nachteile des Standes der Technik besteht die Aufgabe der Erfindung in der Bereitstellung eines Testsystems, welches ein hoch effizientes Screening im HTS-Format erlaubt. Diese Aufgabe wird erfindungsgemäß gelöst durch ein zelluläres Verfahren zur Identifizierung von Substanzen, die fähig sind, die Aktivität eines Zielmoleküls zu beeinflussen, wobei die zu analysierenden Zellen zwei Reportergene tragen, und die Aktivität des Zielmoleküls sich auf die Zellvermehrung auswirkt, mit den Schritten
a. Kontaktierung mindestens einer Zelle mit der zu testenden Substanz,
b. Detektion der Zellvermehrung,
c. Detektion der Aktivität der Reportgenprodukte, dadurch gekennzeichnet, dass ein Reportergen ein Wachstumsmarker und das andere Reportergen ein Enzym, ist, dessen Aktivität anhand der Umsetzung eines Substrates detektierbar ist, wobei das Substrat zeitlich versetzt nach Zugabe der zu analysierenden Substanz zugegeben wird.

Die Detektion von Zellvermehrung und Reportergenaktivität müssen dabei nicht notwendigerweise in obiger Reihenfolge stattfinden.

Das Reportergen kann dabei in das Genom der Zelle integriert oder stabil bzw. transient in die Zelle transfiziert sein. Der Begriff Reportergenprodukt umfasst dabei sowohl die mRNA als auch das Protein. Geeignete Reportergene und deren Produkte sind dem zuständigen Fachmann hinlänglich bekannt, besonders eignen sich hier Enzyme wie β-Galaktosidase, β-Glucuronidase, Luciferase, alkalische Phosphatase, saure Phosphatase oder fluoreszierende Proteine wie GFP, BFP, Aequorin und dergleichen. Geeignete Promotoren für die Reportergene hängen dabei von der Art und Zielsetzung des speziellen Testsystems sowie der verwendeten Zellart ab. Die Reportergene stehen vorzugsweise unter der Kontrolle von Promotoren, die durch den Signaltransduktionsweg reguliert werden, an den das Zielmolekül direkt oder indirekt koppelt. Bevorzugt sind Reportergene, deren Produkte Enzyme sind, deren Aktivität anhand der Umsetzung eines extern zugefügten Substrates detektierbar ist.

Die Zielmoleküle im Rahmen der Erfindung können sich direkt oder indirekt auf die Zellvermehrung der verwendeten Zelle auswirken. Das Zielmolekül kann dabei selbst Einfluss auf die Zellvermehrung der für das Verfahren verwendeten Zelle ausüben (diese also im aktiven Zustand direkt aktivieren oder inhibieren). Das Zielmolekül kann beispielsweise konstitutiv aktiv sein und durch die zu testende Substanz inhibiert werden oder in inaktivem Zustand vorliegen und durch die zu testende Substanz aktiviert werden. Gemäß einer bevorzugten Ausführungsform übt das Zielmolekül den Einfluss auf die Zellvermehrung der für das Verfahren verwendeten Zelle im aktiven Zustand jedoch nur durch Zwischenschaltung eines direkt die Vermehrung beeinflussenden Moleküls aus (z.B. FUS1-HIS3 in Hefezellen, s.u.). Grundsätzlich kommen alle Arten extrazellulärer, membranständiger oder intrazellulärer biologischer Moleküle als Zielmoleküle in Frage, besonders bevorzugt sind humane Biomoleküle, insbesondere Proteine oder Nukleinsäuren, hierunter insbesondere Mitglieder von Signaltransduktionskaskaden der Zellteilung, insbesondere GPCRs, Proteinkinasen, Proteinphosphatasen, etc.

Die Beeinflussung des Zielmoleküls durch die zu analysierende Substanz kann sich dabei auf dessen Aktivität fördernd oder inhibierend auswirken, z.B. durch Interaktion mit dem Zielmolekül selbst oder Beeinflussung von Molekülen, die sich ihrerseits auf die Aktivität bzw. Expression des Zielmoleküls auswirken.

Die Detektion der Zellvermehrung und der Reportergenproduktaktivität kann dabei rein qualitativ aber auch quantitativ erfolgen, verschiedene Arten der Detektion sind dem zuständigen Fachmann hinlänglich bekannt (beispielsweise die Messung der Zelldichte direkt oder indirekt durch die Bestimmung der Trübung von Flüssigkulturen bei Suspensionszellen, colorimetrische oder luminometrische Bestimmungen der Reportergenproduktaktivität etc.).

Gemäß einer bevorzugten Ausführungsform wirkt sich die Aktivität des Zielmoleküls auf die Aktivität, vorzugsweise die Expression des Reportergenproduktes aus. Das Zielmolekül kann sich dabei direkt (das Zielmolekül selbst beeinflusst die Aktivität / Expression des Reportergenproduktes) oder indirekt (das Zielmolekül beeinflusst die Reportergenaktivität bzw. -Expression über eine durch das Zielmolekül aktivierte zelluläre Stoffwechsel- oder Signalkaskade) auf die Aktivität bzw. Expression des Reportergenproduktes auswirken.

Vorzugsweise ist das Zielmolekül ein heterologes Molekül (d. h. nicht natürlich in den für das erfindungsgemäße Verfahren verwendeten Zellen vorhandenes oder exprimiertes Molekül), besonders bevorzugt ein Oligonukleotid, Polynukleotid, eine Nukleinsäure, ein Polypeptid, Protein oder Proteinfragment. Das heterologe Zielmolekül kann dabei in das Genom der Zelle integriert oder stabil bzw. transient in die Zelle transfiziert sein; die Expression des Zielmoleküls kann konstitutiv oder induzierbar sein.

Gemäß einer bevorzugten Ausführungsform übt das heterologe Zielmolekül seine Wirkung auf die Zellvermehrung der für das Verfahren verwendeten Zelle durch Wechselwirkung mit einem chimären Molekül aus. Besonders bevorzugt ist hier ein Verfahren, bei dem das heterologe Zielmolekül ein humanes Molekül ist, welches stabil in das Genom einer nicht humanen Zelle, insbesondere einer Hefezelle, integriert ist, und über ein chimäres Molekül, welches fähig ist, mit dem heterologen Molekül zu interagieren und in die der Hefezelle eigenen Signaltransduktionskaskaden oder Stoffwechselkaskaden zu integrieren, auf die Zellvermehrung Einfluss nimmt. Hierbei ist es besonders bevorzugt, wenn das chimäre Molekül ein rekombinantes Protein, Polypeptid oder Proteinfragment, dessen Aminosäuresequenz humane und Hefeanteile aufweist. Insbesondere eignet sich im Rahmen der Erfindung die Kombination eines humanen GPCRs als heterologes Zielmolekül mit einer chimären G-Protein Untereinheit ("Transplant", s.u.), wobei grundsätzlich jede der Untereinheiten in chimärer Form vorliegen kann.

Zweckmäßig ist es, wenn bei Verwendung der Reportergenprodukte, dessen Aktivität anhand der Umsetzung eines Substrates bestimmt wird, das Substrat zeitlich versetzt nach Zugabe der zu analysierenden Substanz zugegeben wird. Zwischen der Zugabe der zu analysierenden Substanz und der des Substrates liegt dabei vorzugsweise ein Zeitintervall mit der Dauer mindestens eines Zellzyklusdurchlaufs der für das Verfahren verwendeten Zelle, besonders bevorzugt eine Intervall von 2 bis 24 Zellzyklusdurchläufen. Bei Verwendung von Hefezellen beträgt das Zeitintervall vorzugsweise ca. 4 bis 48, vorzugsweise 20 bis 30 und insbesondere 24 Stunden.

Die Detektion der Aktivität des Reportergens erfolgt vorzugsweise unter Aufschluss der Zelle, besonders bevorzugt durch Zugabe einer die Zellwand permeabel machenden oder zerstörenden Substanz (zweckmäßigerweise ein Detergens oder eine Kombination zweier oder mehrer Detergentien; hier eignen sich besonders Digitonin, Triton X-1 00, Nonidet P-40, Tween 20, CHAPS oder SDS). Besonders bevorzugt sind Digitonin im Konzentrationsbereich von 10 bis 600, vorzugsweise 20 bis 400 und besonders bevorzugt von 40 bis 60 µg/ml und/oder Triton X-100 im Konzentrationsbereich von 0,005 bis 0,4, vorzugsweise 0,01 bis 0,2 Vol%, jeweils bezogen auf die Endkonzentration. Die Detergentien werden dem Ansatz dabei vorzugsweise in Pufferlösungen zugesetzt, besonders geeignete Pufferbedingungen sind dem zuständigen Fachmann hinlänglich bekannt (physiologische Puffer, neutraler pH, isotonische Salzkonzentration, etc.).

Für das erfindungsgemäße Verfahren können verschiedenste Arten von Zellen zum Einsatz kommen: So eignen sich grundsätzlich sowohl prokaryontische als auch eukaryontische, pflanzliche oder tierische Zellen. Bevorzugt sind jedoch eukaryontische Zellen, besonders bevorzugt Säugerzellen oder Hefezellen, insbesondere S. cerevisiae Stämme.

Gemäß einer weiteren bevorzugten Ausführungsform der Erfindung werden in einem Ansatz bzw. Verfahrensdurchgang verschiedene Zellen eingesetzt und gleichzeitig gescreent, wobei sich die Zellen mindestens durch die Art des Zielmoleküls voneinander unterscheiden (sogenanntes "Multiplex Verfahren").

Gemäß einer besonders bevorzugten Ausführungsform bezieht sich die Erfindung auf ein breit einsetzbares Verfahren zur Identifizierung von Substanzen, die als Liganden für klonierte G-Protein gekoppelte Rezeptoren wirken. Das erfindungsgemäße Verfahren ist dabei so empfindlich und robust, dass mehrere GPCRs gleichzeitig in einem High-Throughput-Assay im Multiplex-Format getestet werden können.

Die Erfindung wird im folgenden anhand von Ausführungsbeispielen und Figuren näher erläutert.

### Beispiele

### Beispiel 1:

### Screen nach Substanzen, die sich auf die Aktivität G-Protein gekoppelter Rezeptoren auswirken.

Eine der wichtigsten Klassen von Zielmolekülen für die Arzneimittelindustrie stellen G-Protein gekoppelte Rezeptoren dar. Zahlreiche Vertreter dieser Proteinfamilie wurden in der Vergangenheit nach und nach kloniert und pharmakologisch charakterisiert. Da nun das ganze humane Genom sequenziert ist, wurden in letzter Zeit eine große Anzahl von GPCRs auf Sequenzebene identifiziert. Ein Hauptziel der pharmazeutischen Industrie besteht nun darin, Liganden für diese Rezeptoren durch Screening von umfassenden Banken von Substanzen zu identifizieren.

Ungünstigerweise besteht bei den derzeit verfügbaren Methoden und Techniken ein wesentliches Hindernis beim Auffinden von Substanzen in dem hohen Zeit- und Kostenaufwand, der zum Absuchen dieser Banken in Hinblick auf so zahlreiche Zielmoleküle erforderlich ist. EP 0 708 922 B1 legt dar, dass es möglich ist, mehrere GPCRs gleichzeitig in Zellkultur zu screenen. Die dort beschrieben Säugetierzellen, welche GPCRs überexprimieren, reagieren mit einem verstärkten Wachstum, wenn sie mit einer den Rezeptor aktivierenden Substanz in Kontakt gebracht werden. Da die Zellen, die nicht durch diese Substanz aktivierte Rezeptoren exprimieren, trotzdem, wenn auch langsamer, weiterwachsen, ist die Sensitivität des Testsystems nicht sehr hoch. Das Verfahren ist außerdem zeitaufwendig, da die Inkubationszeiten sehr lang sind, und teuer, da es sich um ein Säugetierzellsystem handelt;.

Eine Möglichkeit, kostensparend GPCR zu screenen, bietet der Einsatz eines auf Hefe basierenden Testsystems. Da auch Zeit ein extrem wichtiger Faktor in der pharmazeutischen Forschung ist, bestand das Ziel der vorliegenden Erfindung darin, ein Hefesystem auszuarbeiten, das es ermöglicht, zahlreiche GPCRs gleichzeitig zu screenen. Um den Einsatz im High-Throughput-Screening zu erlauben, sollte das Verfahren möglichst einfach zu handhaben sein und ein möglichst großes Messfenster aufweisen.

G-Protein gekoppelte Rezeptoren (GPCRs) spielen eine wichtige Rolle bei einer Vielzahl physiologischer Prozesse. Sie stellen eine der wichtigsten bisher bekannten Proteinfamilien dar und man nimmt an, dass im menschlichen Genom etwa 1000 Gene für diese Rezeptorklasse kodieren. GPCRs haben eine charakteristische Struktur: Es handelt sich um integrale Membranproteine, die sich siebenfach in Form von □-Helices durch die Phospholipid-Doppelschicht der Zellmembran winden, wobei sie sich kreisförmig anordnen. Man schätzt, dass etwa 50% der gegenwärtig verfügbaren verschreibungspflichtigen Medikamente an GPCRs binden. Dies unterstreicht die bedeutende Rolle dieser Rezeptorklasse für die Pharmaindustrie. Aufgrund der Größe und Bedeutung der Proteinfamilie und angesichts der Tatsache, dass für viele GPCRs noch keine chemischen Bindungspartner bekannt sind (orphan GPCRs), ist davon auszugehen, dass diese Rezeptorklasse in Zukunft eines der wichtigsten Reservoirs für geeignete Zielproteine bei der Suche nach neuen Arzneistoffen sein wird.

Alle G-Protein gekoppelten Rezeptoren funktionieren nach einem gemeinsamen Grundprinzip: Die Bindung eines extrazellulären Liganden führt zur Konformationsänderung des Rezeptorproteins, so dass dieses Kontakt mit einem Guanin-Nukleotid-bindenden Protein (G-Protein) aufnehmen kann. Die an der zytoplasmatischen Seite der Plasmamembran gelegenen G-Proteine sind die Vermittler des extrazellulären Signals in das Zellinnere. Sie lösen in Abhängigkeit von der Spezifität des Rezeptors verschiedene Signaltransduktionswege aus, die alle die Bildung von Botenmolekülen (second messengers), wie beispielsweise cAMP, cGMP, Ca²⁺ oder andere, zur Folge haben, welche über Aktivierung oder Deaktivierung intrazellulärer Proteine Reaktionen in der Zelle auslösen.

Die heterotrimeren G-Proteine bestehen aus drei Untereinheiten, α, β, und γ. Die G□-Untereinheit hat im G-Protein-Heterotrimer GDP gebunden. Die Interaktion mit einem durch einen Liganden aktivierten Rezeptor führt zum Austausch von GDP gegen GTP. Die daraus resultierenden Konformationänderungen führen dazu, dass das G-Protein-Heterotrimer in eine α-Untereinheit und einen βγ-Komplex dissoziiert. Sowohl die aktivierte α-Untereinheit alsauch der βγ-Komplex können intrazelluläre Effektorproteine beeinflussen. Die □-Untereinheiten lassen sich in vier verschiedene Klassen einteilen: Gαs, Gαi, Gαq und Gα12.

GPCRs werden entsprechend des in die Signaltransduktion eingebundenen G-Proteins klassifiziert. D.h. GPCRs der Gs-Familie vermitteln über Aktivierung von Gαs die Stimulation der Adenylatcyclase und erhöhen somit die intrazelluläre cAMP-Konzentration. GPCRs der Gi-Familie vermitteln über Aktivierung von Gαi eine Hemmung der Adenylatcyclase und erniedrigen so die Konzentration intrazellulären cAMPs. GPCRs der Gq-Familie vermitteln über Aktivierung von Gαq eine Stimulation verschiedener PLCβ -Isoformen und führen zu einer Hydrolyse von membranständigen Phosphatidylinosit-4,5-biphosphat zu Diacylglycerol und Inositoltriphosphat (IP₃). IP₃ setzt aus intrazellulären Speichern Ca²⁺ frei. Gα12 interagiert mit rho-spezifischen Guanin-Nukleotid-Austauschfaktoren.

Das Signal hält so lange an bis die Gα-Untereinheit, welche ein GTPasen-Aktivität besitzt, das gebundene GTP hydrolysiert. Mitglieder der Familie der RGS-Proteine (regulator of G protein signalling) steuern die zeitliche Dauer des Signals, indem sie als Aktivatoren auf die GTPasen-Aktivität der Gα-Untereinheit wirken. Dieses von G-Proteinen gesteuerte Signaltransduktionssystem scheint allen eukaryontischen Systemen gemeinsam zu sein.

Ein sehr gut charakterisiertes Beispiel für ein solches Signalsystem stellt der sogenannte "Pheromone Response Pathway" der Bäckerhefe Saccharomyces cerevisiae dar. Hefezellen mit dem MATa Mating Typ exprimieren einen Rezeptor, welcher vom STE2 Gen kodiert wird. Dieser Rezeptor wird aktiviert durch die Bindung von α-Faktor, einem Peptidpheromon, welches von Hefezellen des anderen Mating Typs (MATα) freigesetzt wird. Das heterotrimere G-Protein der Hefe setzt sich aus den Produkten der Gene GPA1 (Gα), STE4 (Gβ) und STE18 (Gγ) zusammen. Der Gβγ-Komplex wird nach der Aktivierung des Ste2p Rezeptors freigesetzt und überträgt das Signal auf eine Mitogen-aktivierte Proteinkinasekaskade. Dies führt zur Aktivierung des Cyclin-abhängigen Kinaseinhibitors Far1p, was einen Arrest in Zellzyklus und die transkriptionelle Induktion einer Reihe von Genen, die in den Mating-Prozess involviert sind (z.B. FUS1), zur Folge hat. Dieser Pathway wird von Sst2p, einem Mitglied der RGS-Familie, desensibilisiert. Hefezellen des anderen Mating Typs (MATα) exprimieren einen anderen Rezeptor (Ste3p) und antworten daher auf das andere Pheromon (a-Faktor), das von MATa-Zellen freigesetzt wird. Abgesehen davon ist der Signalapparat, den die zwei Mating Typen verwenden, der gleiche.

Es wurde mehrfach gezeigt, dass Säugetier-GPCRs an das G-Protein-Signalsystem der Hefe gekoppelt werden können. Einige Rezeptoren, einschließlich des Ratten Somatostatin 2 Rezeptors (Price et al., Mol Cell Biol 15, 6188-6195 (1995)) und des Ratten Adenosin A₂ₐ Rezeptors (Price et al., Molecular Pharmacology 50, 829-837 (1996)), können direkt mit dem Hefe-Gα-Protein Gpa1p interagieren, wogegen andere Rezeptoren, einschließlich des Growth-Hormone-Releasing-Hormone Rezeptors (GHRHR) (Kajkowski et al., J Recept Signal Transduct Res 17, 293-303 (1997)), inkompatibel zu Gpa1p sind. Um trotzdem ein Koppeln dieser Rezeptoren zu ermöglichen, kann die Hefe-Gα-Untereinheit deletiert werden und der heterologe Rezeptor wird dafür zusammen mit der Volllänge-Säugetier-Gα-Untereinheit exprimiert. Alternativ dazu wurden Hybrid-Gα-Untereinheiten verwendet, bei denen die C-terminale Domäne (ungefähr ein Drittel der Peptidsequenz) von Gpa1p mit durch die entsprechende Region der Säugetier-Gα-Untereinheit ersetzt wurde. Siehe WO95/21925 für beide Ansätze. Hybride oder andere modifizierte oder heterologe Gα-Untereinheiten müssen einige Kriterien erfüllen, um an das Hefe-Signaltransduktionssystem koppeln zu können. Am wichtigsten ist, dass sie einerseits effizient an Hefe Gβγ binden können, um so ein Signal in der Abwesenheit eines aktivierten GPCRs verhindern zu können, und andererseits effektiv an den durch einen Agonisten aktivierten Rezeptor binden können, um dann das Signal weiterleiten zu können. Conklin et al., Nature 363, 274-276 (1993) beschrieb zum ersten Mal ein Hybrid, bei dem die C-terminalen 5 Aminosäuren von Gαq durch die entsprechende Gαi-Sequenz ersetzt worden ist (GαqiS), was eine Umkopplung von normalerweise Gαi gekoppelte Rezeptoren and den Gaq-Signaltransduktionsweg ermöglichte. In WO99/14344 und Brown et al., Yeast 16, 11-22 (2000) wird dargelegt, dass der gleiche Ansatz auch in Hefe funktioniert. In diesem Fall wurden die C-terminalen 5 Aminosäuren von Gpa1p durch die entsprechenden Aminosäuren aller humaner Gα-Proteine ersetzt. Der Einsatz dieser "Transplants" genannter Hybride ermöglicht das Koppeln zahlreicher Säugetier-GPCRs and den Mating Pathway der Hefe.

Die hier eingesetzten Hefestämme tragen Deletionen in den SST2, FAR1 und, je nach Mating Typ der Zelle, STE2 oder STE3 Genen. SST2, ein Mitglied der Familie der RGS-Proteine, ist deletiert, um die Runterregulierung des Signals zu verhindern. Die Deletion von FAR1 ermöglicht, dass das Zellwachstum auch unter Bedingungen weitergeht, unter denen der Pheromone Response Pathway angeschaltet ist. STE2 oder STE3 wird ausgeschaltet, um eine unerwünschte Kompetition um das heterotrimere G-Protein zu verhindern. Das GPA1 Gen wurde im Hefegenom durch die oben beschriebenen Transplants ersetzt. Durch die Expression der Transplants unter der Kontrolle des GPA1-Promotors am natürlichen Genlocus wird sichergestellt, dass die Stöchiometrie des heterotrimeren G-Proteins erhalten bleibt.

Die Modifizierung der Wirkung wenigstens eines von einem GPCR abhängigen Signaltransduktionswegs eines biologischen Organismus kann inhibierend oder stimulierend erfolgen. Eine inhibierende Wirkung einer chemischen Verbindung liegt vor, wenn das vom Signaltransduktionsweg abhängige messbare Signal in Anwesenheit einer chemischen Verbindung schwächer ausfällt, als wenn dieses wegegelassen wird. Verbindungen, die eine solche Wirkung hervorrufen werden auch Antagonisten genannt. Andererseits liegt eine stimulierende Wirkung einer chemischen Verbindung vor, wenn das vom Signaltransduktionsweg abhängige messbare Signal stärker ausfällt, als wenn die chemische Verbindung weggelassen wird. Solche Verbindungen werden auch Agonisten genannt.

Für funktionelle Assays in Saccharomyces cerevisiae ist die Verwendung der Promotoren der Gene FUS1, FUS2 (Cismowski et al., Nat Biotechnol 17, 878-883 (1999); Frederickson, Nat Biotechnol 17, 852-853 (1999)) und YNL279w (WO 02/40660) beschrieben. Diese Gene werden als Antwort auf eine Stimulierung des Pheromone Response Pathways durch Mating Faktor erhöht exprimiert. Wird nun ein Promotorelement eines dieser Gene funktionell mit einem Strukturgen verbunden, so kann die Expression dieses Strukturgens (auch Reportergen genannt) über den beschriebenen Signaltransduktionsweg der Hefe reguliert werden. Solche Reportergene sind normalerweise endogene Wachstumsmarker wie HIS3 oder andere auxotrophe Markergene (z.B. URA3, LEU2, ADE2, LYS1 oder TRP1), welche bei stimuliertem Signaltransduktionsweg ein Wachstum der Zellen in einem entsprechend depletiertem Medium zulässt, oder Gene, die Resistenz oder Sensitivität gegenüber bestimmten Substanzen verleihen (z.B. CYH2 oder G418^{R}). Es können aber auch Reportergene verwendet werden, die für intrazelluläre Enzyme, wie β-Galaktosidase (LacZ), oder "Green Fluorescent Protein" (GFP) oder für sezernierte Enzyme, wie Phosphatasen (PHO5), kodieren. Wird als Reporter CAN1 verwendet, so wachsen die Zellen in Canavanin-haltigem Medium. In Gegenwart von Aktivatoren (Agonisten) eines heterolog exprimierten GPCRs wird das CAN1-Gen exprimiert, sodass die Zellen nicht mehr in Canavanin-haltigem Medium wachsen. Wird ein Inhibitor (Antagonist) hinzugefügt, so wachsen die Kulturen in diesem Selektionsmedium.

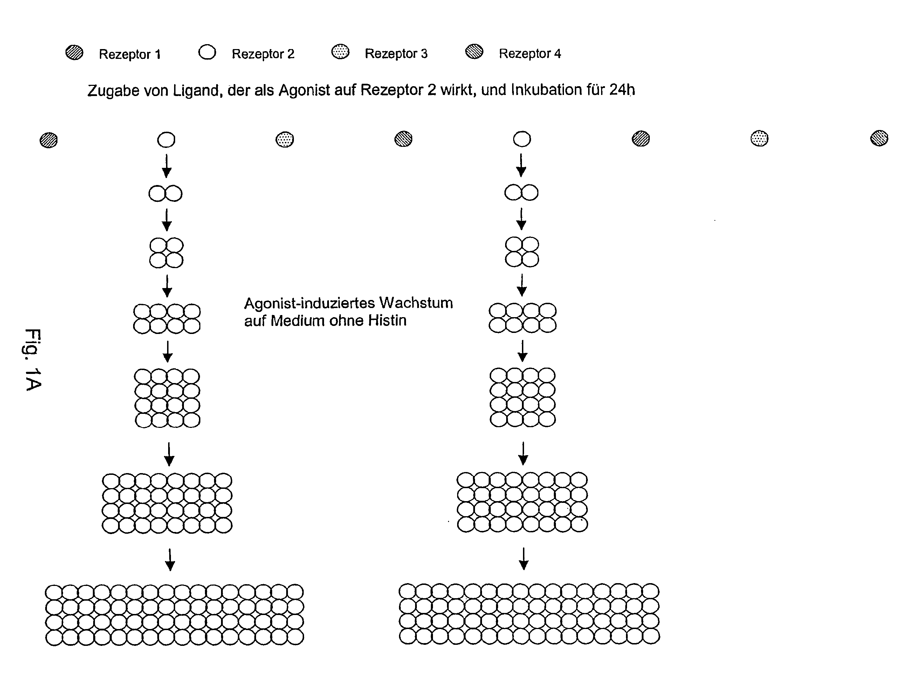

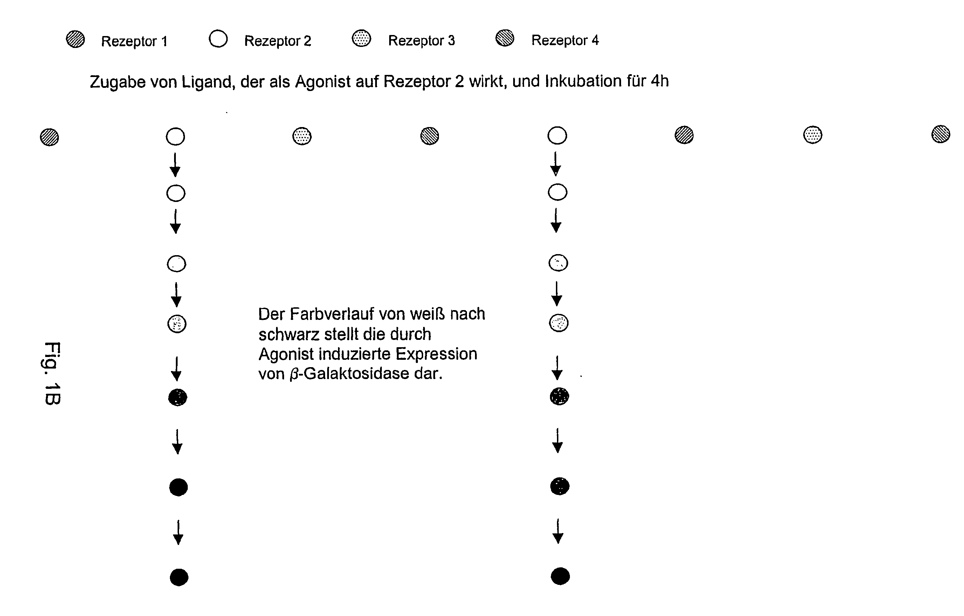

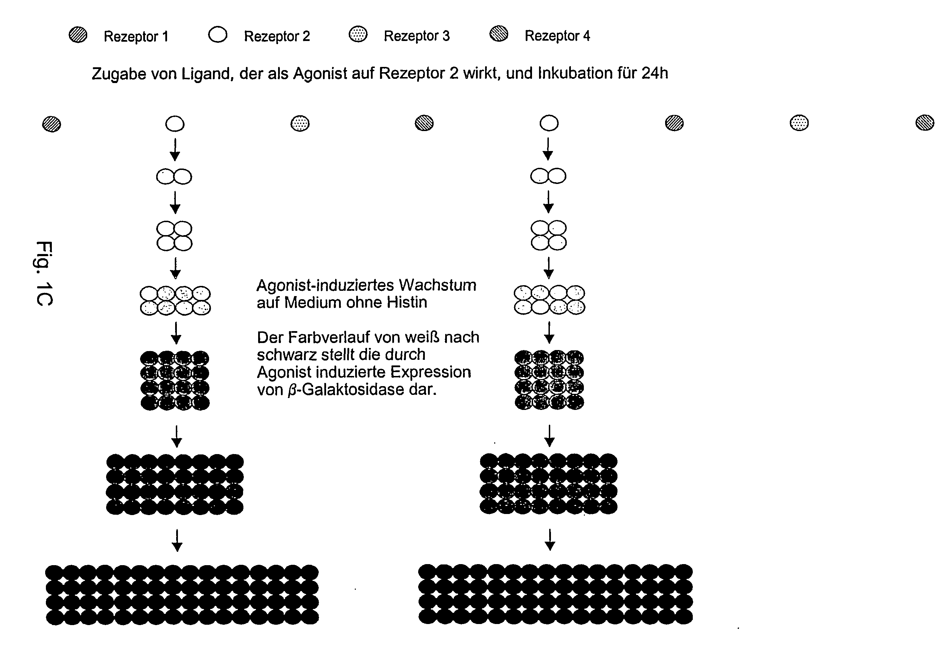

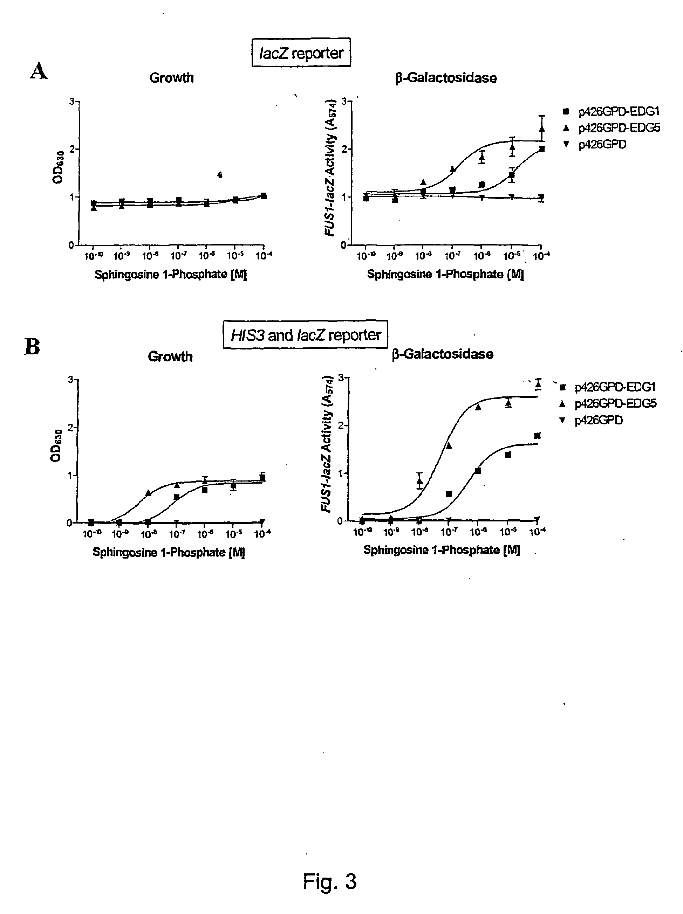

Die in der Literatur beschriebenen Hefe-GPCR-Assays benutzen meist nur ein Reportergen, meist HIS3 oder LacZ (Price et al., Mol Cell Biol 15, 6188-6195 (1995); Price et al., Molecular Pharmacology 50, 829-837 (1996); Campbell et al., Bioorg.Med.Chem.Lett. 9, 2413-2418 (1999); Pausch, Trends Biotechnol 15, 487-494 (1997)) unter der Kontrolle des FUS1-Promotors (FUS1-HIS3 oder FUSI-lacZ). Wird FUS1-HIS3 verwendet, so wird eine Aktivierung des Signaltransduktionsweg als Trübung der Hefekultur in einem Flüssigmedium ohne Histidin gemessen. Experimente der Erfinder zeigten, dass der alleinige Wachstums-Read-out ein Signal-zu-Hintergrund-Verhältnis von ca. 30-50:1 in Flüssigkultur liefert (s. Figuren 1a und 3). β-Galaktosidase-Flüssigassays unter Verwendung von Chlorphenolred β-D-Galaktopyranosid (CPRG) als Enzymsubstrat zeigten nach Stimulierung ein ca. 2-3-fach über Hintergrund erhöhtes Signal (s. a. Figur 1b). Im Bemühen das Messfenster weiter zu vergrößern, wurden beide Reportergene gleichzeitig in einer Hefezelle eingesetzt, da dies zu einer Multiplikation der beiden Messsignale führen sollte. In Fig. 1c ist dieses Prinzip zur Verdeutlichung dargestellt. Dieser Doppel Reportergen-Assay führte dann auch zu einer Verbesserung des Signal-zu-Hintergrund-Verhältnisses auf ca. 100-150:1. Brown et al., Yeast 16, 11-22 (2000), beschreibt einen ähnlichen Assay. Auch hier werden FUS1-HIS3 und FUS1-IacZ gleichzeitig in einem β-Galaktosidase-Flüssigassay unter Verwendung von CPRG als Substrat benutzt. CPRG wird hier während der gesamten Zeitdauer der Stimulierung des Rezeptors mit Ligand zugesetzt. Im Unterschied dazu wird in diesem Verfahren CPRG zusammen mit einem Detergenz in gepufferter Lösung erst nach der Stimulierung des Rezeptors mit Ligand zugegeben, was zu einer deutlichen Verbessung des β-Galaktosidase-Messwertes führt. Ist nämlich zum einen CPRG während des vom Liganden induzierten Wachstums anwesend, wird dieses leicht inhibiert, zum anderen kann CPRG nur schlecht durch die Plasmamembran ins Innere der Zelle gelangen. Beide Probleme werden umgangen, wenn CPRG erst nach erfolgtem Wachstum zusammen mit einem Detergenz, welches die Plasmamembran aufbrechen kann, zugegeben wird.

Das Verfahren bedient sich in einer bevorzugten Ausführungsweise eines Doppel-Reportergen-Assays, wobei das eine Reportergen ein Wachstumsmarker und das andere Reportergen ein Enzym oder GFP ist. Nur diese Kombination von Wachstum, einem logarithmischen Ereignis, und der mehr oder wenigen linear induzierten Expression eines messbaren Enzyms oder fluoreszierenden Proteins, führt zu der beschriebenen Amplifikation des Signals, d.h. einem großen Messfenster.

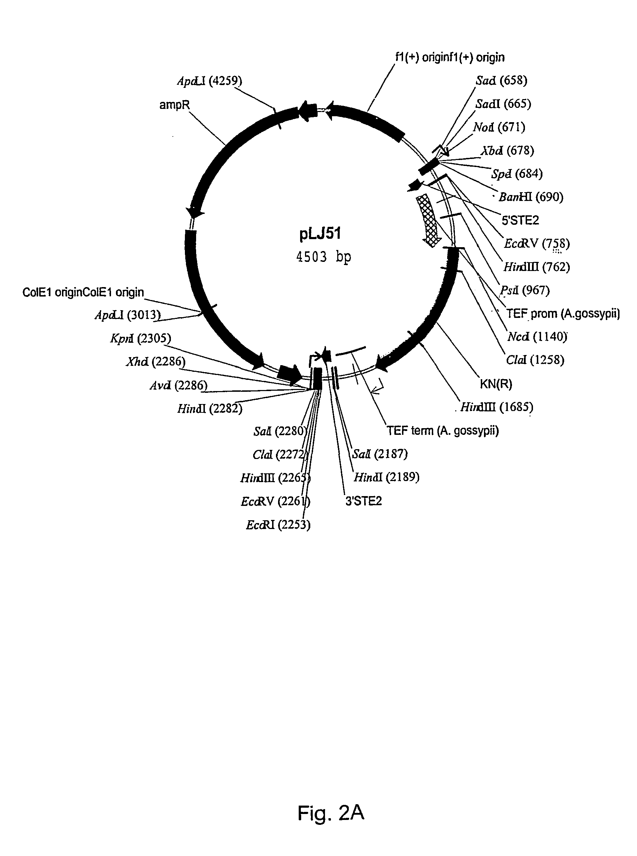

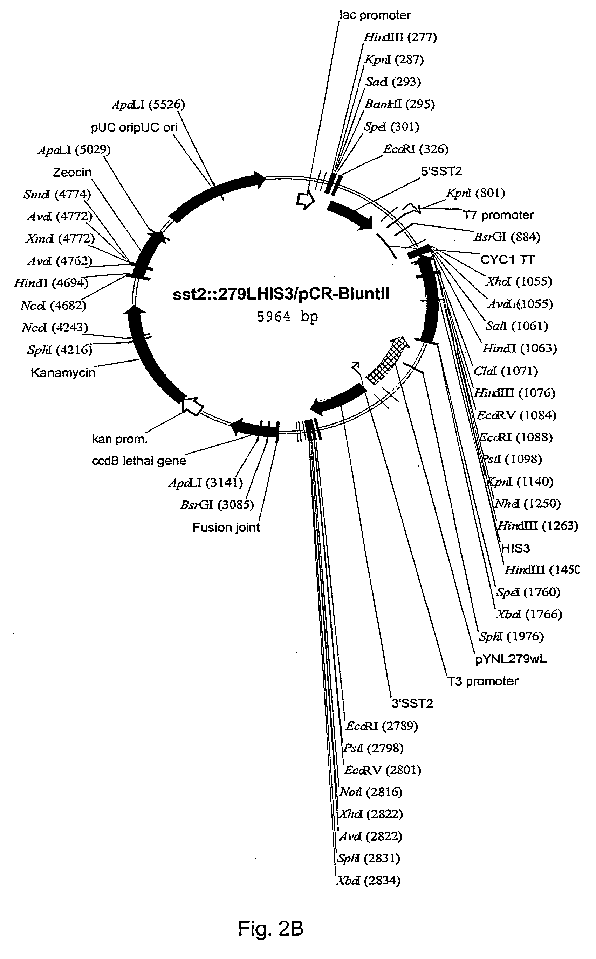

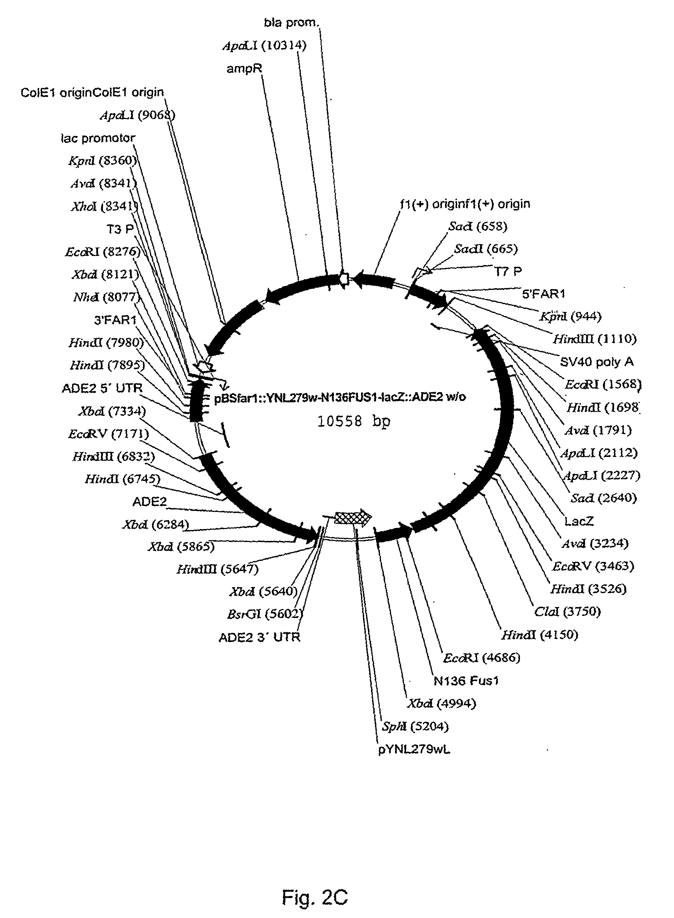

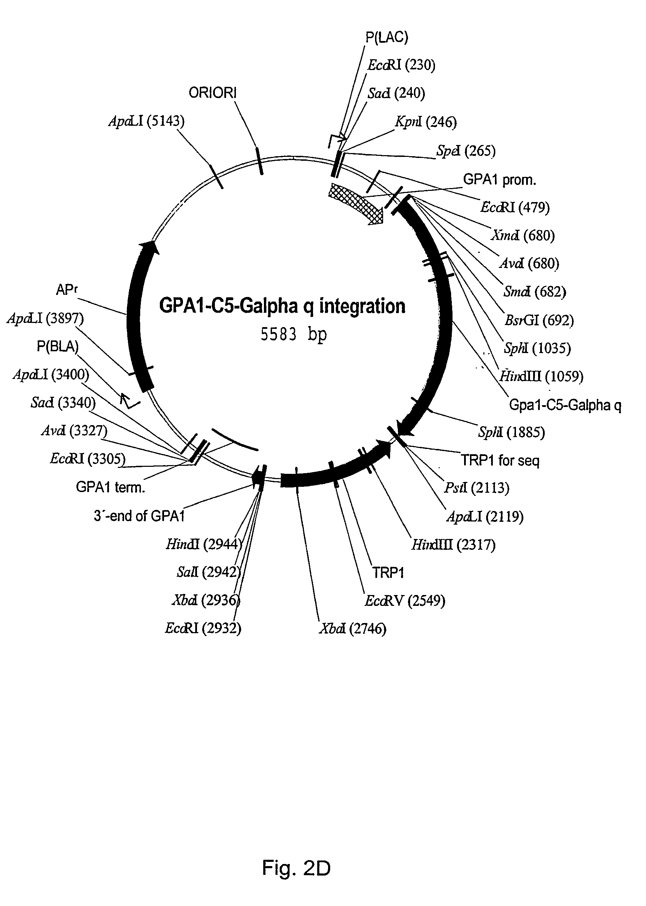

EP 0 708 922 B1 (Acadia Pharmaceuticals) beschreibt auch ein Verfahren, dass auf Wachstum als Antwort auf Rezeptorstimulation beruht. Hierbei wachsen die durch Ligand stimulierten Rezeptor-exprimierenden Zellen aber nur schneller als die nicht stimulierten Zellen (vgl. Fig. 2 und Fig. 10 in EP 0 708 922 B1). In der hier beschriebenen Erfindung, wachsen solche nicht stimulierten Hefezellen aber gar nicht (Siehe angeführte Beispiele (z.B. Fig. 3B, linker Graph). EP 0 708 922 B1 verwendet als messbares Signal auch die Aktivität des heterolog exprimierten Enzyms β-Galaktosidase. LacZ wird hier aber konstitutiv exprimiert, d.h. die gemessene Enzymaktivität ist nur ein Maß für die Anzahl der Zellen, die als Antwort auf Stimulierung des Signaltransduktionsweges durch Ligand gewachsen sind, nicht aber ein Maß für die Stärke der Stimulierung des Signaltransduktionsweges. In der hier beschriebenen Erfindung steht die Expression von LacZ in Gegensatz dazu unter der Kontrolle eines durch den Pheromone Response Pathway induzierten Promotors (z.B. FUS1 oder YNL279w). In Fig. 3A ist dargestellt, dass auch bei gleicher Anzahl von Hefezellen (linker Graph) die messbare Aktivität der β-Galaktosidase von der zugesetzten Menge an Ligand, d.h. von der Stärke der Stimulierung des Signaltransduktionsweges, abhängt (rechter Graph).

Unter "Amplifikation" von Zellen wird laut EP 0 708 922 B1 (vgl. S. 10) "das Wachstum von mit Rezeptor transfizierten Zellen, insbesondere im Vergleich zum Wachstum von nicht mit dem Rezeptor transfizierten Zellen" verstanden. D.h. sowohl mit Rezeptor transfizierte als auch nicht transfizierte Zellen können wachsen, nur dass die transfizierten Zellen nach Stimulierung mit Ligand schneller wachsen. Dies wird in den Abbildungen auf S. 33 und S.44 verdeutlicht. In den beschriebenen Zelllinien ist kein Reporterkonstrukt vorhanden, dessen Expression erst das Wachstum ermöglicht. Die einzige Modifikation, die das Wachstum ermöglicht, ist der durch Ligand stimulierte überexprimierte Rezeptor.

Gemäß der bevorzugten Ausführungsform wird aber eine doppelte Selektion betrieben:
Dem Nährmedium fehlen Uracil und Histidin, Substanzen, die die hier verwendeten Hefestämme zum Leben braucht. Da wir das URA3-Gen als selektiven Marker auf unseren Rezeptorplasmiden verwenden, können Zellen, denen das Rezeptorplasmid fehlt, überhaupt nicht wachsen.
Auch die mit Rezeptor-DNA transfizierte Zellen können grundsätzlich so lange auf diesem Nährmedium nicht wachsen, solange sie nicht durch die Gegenwart von Ligand stimuliert werden. Erst wenn der Ligand bindet, wird das Reportergen HIS3 exprimiert und die Zellen sind in der Lage auf dem Nährmedium zu wachsen. Grundsätzlich reagiert ein Hefestamm, der mit einem Rezeptor transfiziert ist, aber kein Wachstumsmarker wie HIS3 als Reportergen trägt, nicht mit Wachstum.

Das beschriebene Verfahren kann sowohl im Einzelrezeptor-Format als auch im Mehrfachrezeptor-Format (Multiplex-Format) eingesetzt werden. Besonders im Mehrfachrezeptor-Format kommen die Vorteile dieses Assays zum Tragen. Fig. 1c soll dies verdeutlichen. Theoretisch sollte eine Hefezelle, die einen bestimmten Rezeptor exprimiert, ausreichen, dass wenn diese Zelle mit dem passenden Liganden (einer chemischen Verbindung oder dem natürlichen Liganden) in Kontakt kommt, als Antwort auf die Stimulierung sozusagen aus dem Hintergrund der nicht reagierenden anderen Hefezellen "herauswächst". Dies bringt Vorteile für das High-Throughput-Screening, da es diese Methode erlaubt mehrere GPCRs gleichzeitig zu screenen. Gerade für orphan GPCRs, deren Bedeutung für die pharmazeutische Industrie von vorne herein nicht klar ist, minimiert die hier beschriebene Methode den zeitlichen und finanziellen Einsatz des Unternehmens. Da von orphan GPCRs auch nicht bekannt ist, an welche Gα-Untereinheit sie koppeln, besteht mit dieser Methode auch die Möglichkeit einen oder mehrere orphan GPCRs in mehreren Transplant-Stämmen gleichzeitig zu testen.

### Beschreibung der Figuren

Fig. 1 verdeutlicht das Prinzip des Doppel-Reportergen-Assays im Mehrfachrezeptor-Format: Figur 1a zeigt einen Agonisten-induzierten Wachstums Read Out. Figur 1b stellt einen Agonisten-induzieren β-Galaktosidase vermittelten Farb Read Out dar. Figur 1c. schließlich zeigt den doppelten Agonisten-induzierten Wachstums und Farb Read Out.
Fig. 2A-D zeigen Plasmide, die zur Konstruktion der Stämme verwendet wurden, die auf dem YNL279w-Promotor basieren.
In Fig. 3 ist dargestellt, wie der Doppel-Reportergen-Assay die Leistungsfähigkeit des Hefe-Flüssigassays verbessert, verglichen zur Nutzung nur eines Reportergens.
Fig. 4 zeigt die Anbindung des humanen Bradykinin B2 Rezeptors an den Hefe-Signaltransduktionsweg in Abhängigkeit vom eingesetzten Gα-Transplant. Als Kontrollen wurde immer der leere Vektor p426GPD verwendet.
Fig. 5 zeigt, dass der Doppel-Reportergen-Assay auch zum Screening von Antagonisten eingesetzt werden kann. Als Beispiel sind der humanen Bradykinin B2 Rezeptors bzw. die leere Vektorkontrolle dargestellt.
In Fig. 6A und B wird gezeigt, dass die Verwendung des YNL279w-Promotors auch nach 29h Inkubation mit dem Enzymsubstrat deutlich weniger Hintergrundsignal verursacht als die Verwendung des FUS1-Promotors.
Fig. 7 erklärt die Durchführung eines Assays im Mehrfachrezeptor-Format. In Fig. 7A wird deutlich gemacht, dass die verschiedenen GPCRs jeweils in einem eigenen Hefestamm exprimiert werden, nicht alle zusammen in einem. Fig. 7B zeigt die Leistungsfähigkeit eines Assays im Mehrfachrezeptor-Format im Vergleich zum Einfachrezeptor-Format in einer Mikrotiterplatte.
Fig. 8 zeigt, dass die Leistungsfähigkeit des Assays steigt, wenn das Enzymsubstrat CPRG zusammen mit Detergenz erst nach der Inkubation mit Liganden zugesetzt wird.

### Material und Methoden

### Plasmide und Hefegenetik:

Alle molekularbiologischen und genetischen Manipulationen wurden nach Standardmethoden durchgeführt (Ausubel et al., Current Protocols in Moleculat Biology, Wiley & Sons, New York; Guthrie and Fink, Guide to Yeast Genetics and Molecular Biology, Methods in Enzymology, Academic Press, San Diego).

### Expressionskonstrukte für Rezeptoren:

Alle Expressionskonstrukte basieren auf dem episomalen 2□-Hefe-E. coli-Shuttle-Vektor p426GPD (Mumberg et al., Gene 156, 119-122 (1995)). cDNA-Sequenzen, welche für die humanen G-Protein gekoppelten Rezeptoren kodieren, werden in diesem Vektor zwischen dem GPD-Promotor und dem CYC-Terminator kloniert, um eine starke konstitutive Expression in Hefezellen zu erreichen. Die folgenden humanen GPCRs wurden in diesen Vektor kloniert:
EDG1 Rezeptor (Genbank NM_001400), EDG5 Rezeptor (Genbank NM_004230), Bradykinin B2 Rezeptor (Genbank NM_000623), M1 Muskarinrezeptor (Genbank NM_000738), Somatostatin SSTR2 Rezeptor (Genbank NM_001050). M3 Muskarinrezeptor (Genbank NM_000740)

### Hefestämme:

Alle Hefestämme beruhen auf dem Saccharomyces cerevisiae Wildtypstamm W303-1a, beschrieben unter der ATCC-Nummer 208352.

Genotyp: MATa, ade2-1, ura3-1, his3-11, trp1-1, leu2-3, leu2-112, can1-100

Es wurden zwei verschiedene Sets von Hefestämmen eingesetzt. Ein Set stammt von YLJ21 ab und benutzt den Promotor des FUS1 Gens zur Expression der Reportergene, das andere Set stammt von YSG13 ab und nutzt den Promotor des YNL279w Gens.

Der Hefestamm YLJ21 wurde von Ekkehard Leberer zur Verfügung gestellt.

Genotyp: MATa, ste2::KanR sst2::ura3^{FOA} far1::hisG FUS1::HIS3 mfa2::FUS1-lacZ::ura3^{FOA} ade2-1, ura3-1, his3-11, trp1-1, leu2-3, leu2-112, can1-100

Die Aktivierung des Pheromone Response Pathways kann mit Hilfe der zwei Reportergene FUS1::HIS3 und FUS1-IacZ gemessen werden, welche am HIS3- bzw. MFA2-Genlocus integriert wurden. Das FAR1 Gen wurde durch ein hisG-Repeat ersetzt, sodass die Zellen weiterwachsen können, auch wenn der Pheromone Response Pathway aktiviert ist. Das SST2 Gen wurde durch das URA3 Gen ersetzt, um die Runterregulierung des G-Protein-Signals durch die GTPasen-Funktion von Sst2p zu verhindern. Die ura3-Marker wurde jeweils durch Selektion auf 5-Fluoroorotic-acid-haltigem Medium wieder zurückgewonnen. Das Gen STE2, das für den α-Faktor Rezeptor kodiert, wurde durch ein KanR Gen ersetzt.

### Der Hefestamm YSG13 wurde folgendermaßen hergestellt:

Genotyp:
   MATa, ste2::KanR sst2::pYNL279w-H1S3 far1::pYNL279w-N136FUS1-IacZ::ADE2 ade2-1, ura3-1, his3-11, trpl-1, leu2-3, leu2-112, canl-100
Stammkonstruktion:
   ste2::KanR
      Um das Hefe STE2-Gen durch ein Kanamycin-Resistenzgen zu ersetzen wurde das Plasmid pLJ51 mit BamH1 und EcoR1 geschnitten und in den Wildtyp-Hefestamm W303-1 a transformiert. Selektion erfolgte auf YPD+G418-Medium.
   sst2::pYNL279w-H1S3

In einem weiteren Schritt wurde das Hefe SST2 Gen durch eine Kassette ersetzt, welche die Expression des HIS3 Gens unter der Kontrolle des YNL279w-Promotors erlaubt. Dazu wurde das Plasmid sst2::279LHIS3/pCR-Bluntll mit BamHI und Notl geschnitten und transformiert. Selektion erfolgte auf SC/Gluc-His+α-Faktor-Medium. far1::pYNL279w-N136FUS1-IacZ::ADE2

Anschließend wurde das FAR1 Gen durch eine Kassette ersetzt, welche die Expression der N-terminalen 136 Aminosäuren von Fus1p fusioniert an β-Galaktosidase unter der Kontrolle des YNL279w-Promotors erlaubt. Dazu wurde das Plasmid pBSfar1::YNL279w-N136FUS1-IacZ::ADE2 w/o mit Sacll und Xhol geschnitten und transformiert. Selektion erfolgte auf SC/Gluc-Ade-Medium.

Die korrekte Integration aller Fragmente ins Genom wurde immer mittels PCR überprüft.

### Einfügen der Transplants in YLJ21 und YSG13::

Ausgehend von den Stämmen YLJ21 und YSG13 wurden schließlich die letzten 5 Aminosäuren der Hefe G-Protein-α-Untereinheit Gpa1 im Hefegenom durch die letzten 5 Aminosäuren der humanen G-Protein-α-Untereinheiten ersetzt. Dazu wurde zum Beispiel zur Konstruktion des Hefestammes YEW3 das Plasmid GPA1-C5-Galpha q integration mit Sacl geschnitten und in Hefestamm YLJ21 transformiert. Selektion erfolgte auf SC/Gluc-Trp-Medium. Die Integration der anderen Transplants erfolgte auf die gleiche Weise. In Tabelle 1 sind die verschiedenen Transplants und die davon abgeleiteten Hefestämme aufgeführt.

**Tabelle 1: G-Protein Transplants**

| GPA1/GαX Transplant | steht für humane G-Protein α-Untereinheit | C-terminale 5 Aminosäuren | FUS1-Promotor | YNL279w-Promotor |
|---|---|---|---|---|
| GPA1 | - | KIGII | YLJ21 | YSG13 |
| i1 | t, i1, i2 | DCGLF | YEW11 | YEW25 |
| i3 | i3 | ECGLY | YEW7 | YEW21 |
| o | o1, o2 | GCGLY | YEW8 | YEW22 |
| z | z | YIGLC | YEW12 | YEW26 |
| q | q, 11 | EYNLV | YEW3 | YEW17 |
| 14 | 14 | ENFLV | YEW6 | YEW20 |
| 16 | 15, 16 | EINLL | YEW2 | YEW16 |
| 12 | 12 | DIMLQ | YEW13 | YEW27 |
| 13 | 13 | QLMLQ | YEW14 | YEW28 |
| s | s1, s2 | QYELL | YEW1 | YEW15 |

### Doppel-Reportergen-Assay:

Die in den Vektor p426GPD klonierten humanen GPCRs werden in den jeweiligen Hefestamm transformiert und auf SC-Selektionsplatten ohne Uracil und mit 2% Glukose als Kohlenstoffquelle (SC/Gluc-Ura) für 3 Tage bei 30°C inkubiert. Die so erhaltenen Einzelkolonien werden dann benutzt, um 2ml-Übernachtkulturen in SC/Gluc-Ura anzuimpfen. Am nächsten Tag werden die Zellen 1:100 in SC/Gluc-Ura-His pH6.8 verdünnt. Im Fall von Hefestämmen, die den FUS1-Promotor zur Reportergenexpression verwenden, wird diesem Medium noch 2-10mM 3-Aminotriazol (3-AT, Sigma) zugesetzt. Je 90µl der verdünnten Zellsuspension werden in eine Vertiefung einer Mikrotiterplatte mit 96 Vertiefungen pipettiert, in der sich bereits 10µl des zu untersuchenden Liganden befinden. Die Platten werden mit oder ohne Schütteln für 5-24h bei 30°C inkubiert. Dann wird je Vertiefung 50µl Assay Mix zugegeben. Der Assay Mix besteht aus 150µg/ml Digitonin (Sigma), 300µg/ml Chlorphenolred-β-D-Galaktopyranosid (CPRG, Roche), 300mM Natriumphosphat-Buffer pH6.7. Nach Inkubation für 2h bei 30°C mit oder ohne Schütteln wird die β-Galaktosidase-Aktivität als Absorption bei 574nm in einen Spektrophotometer (Spectramax Plus, Molecular Devices) gemessen. Die Auswertung der Daten und das Zeichnen der Dosis-Wirkungskurven erfolgt mit dem Graphpad Prism 3.0 Computerprogramm. Alle Messpunkte sind Mittelwerte aus Dreifachbestimmungen.

### Beispiel2:

### Vergleich zwischen dem Einsatz von einem Reportengen allein oder zwei Reportergenen gleichzeitig

Der Hefestamm YLJ21 wurde entweder mit dem leeren Vektor p426GPD oder den humanen GPCRs EDG1 und EDG5, welche in p426GPD kloniert waren, transformiert. Die transformierten Hefen wurden dann in 2ml SC/Gluc-Ura über Nacht bei 30°C angezüchtet. Am nächsten Tag wurden die Kulturen 1:100 in SC/Gluc-Ura-His pH6.8-Medium ohne (Fig. 3A) oder mit 2mM 3-AT (Fig. 3B) verdünnt. Je 90µl der verdünnten Zellsuspension werden in eine Vertiefung einer Mikrotiterplatte mit 96 Vertiefungen pipettiert, in der sich bereits je 10µl einer Serienverdünnung von Sphingosin-1-phosphat (Biomol) oder als Kontrolle reines Wasser befinden. Die Platten werden für 23h bei 30°C unter Schütteln (700 U/min) inkubiert. Die Trübung, welche aus dem Wachstum der Hefezellen resultierte, wurde bei 630nm im Photometer gemessen (Fig. 3A und B jeweils linker Graph). Danach wurde 50µl Assay Mix je Vertiefung zugegeben. Nach 2h Inkubation bei 30°C unter Schütteln wurde die β-Galaktosidase-Aktivität als Absorption bei 574nm im Photometer gemessen.

Der linke Graph von Fig. 3A zeigt, dass bei Verwendung von FUS1-HIS als Reporterkonstrukt ohne die Zugabe von 3-AT keine Dosis-Wirkungskurve zu erkennen ist. Der FUS1-Promotor führt auch ohne Stimulierung des Signaltransduktionsweges zu einem recht hohen Hintergrundsignal auf Medium ohne Histidin, d.h. der Promotor ist nicht strikt reguliert. Wird dagegen 2mM 3-AT, ein kompetitiver Inhibitor von His3p, zugesetzt, so wird das Hintergrundsignal unterdrückt und die Höhe des Messsignals ist abhängig von der Menge an Sphingosin-1-phosphat (Ancellin et al., J Biol Chem 274, 18997-19002 (1999)) im Medium, wenn EDG1 oder EDG5 exprimiert werden (Fig. 3B, linker Graph). Aus Fig. 3A rechter Graph ist ersichtlich, dass LacZ als Reportergen auch zu einer akzeptablen Dosis-Wirkungkurve führt, das Messfenster ist jedoch sehr klein. Wie Fig. 3B rechter Graph zeigt, führt der Einsatz von HIS3 und LacZ gleichzeitig zu einem vielfach besserem Signal-zu-Hintergrund-Verhältnis. Insgesamt zeigt dieses Experiment, dass die humanen GPCRs EDG1 und EDG5 durch die Hefeeigene Gα-Untereinheit Gpa1p an den Pheromone Response Pathway koppeln können.

### Beispiel 3:

### Doppel-Reportergen-Assay unter Anbindung des humanen Bradykinin B2 Rezeptors an den Signaltransduktionsweg der Hefe durch Gα-Transplants

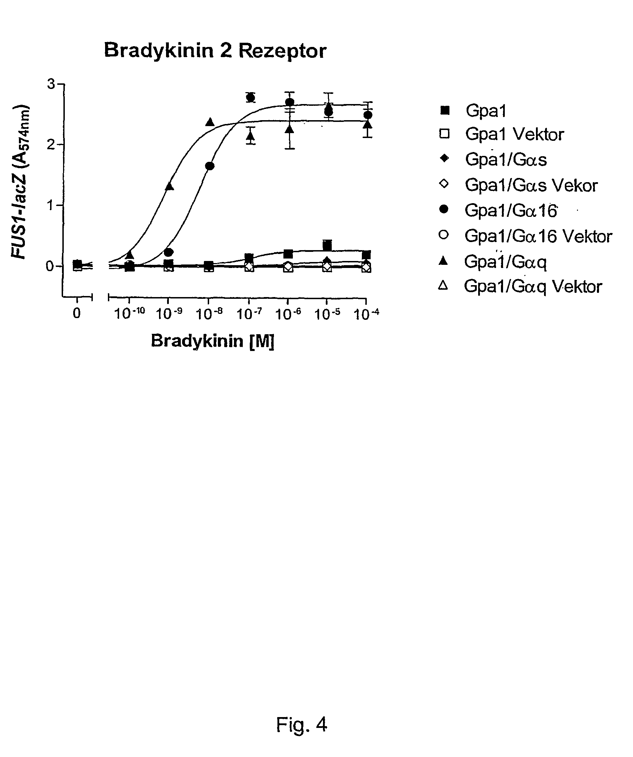

Die Hefestämme YLJ21 (Gpa1), YEW1 (Gpa1/Gαs), YEW2 (Gpa1/Gα16) und YEW3 (Gpa1/Gαq) wurden entweder mit dem leeren Vektor p426GPD oder dem humanen Bradykinin B2 Rezeptor, der in p426GPD kloniert war, transformiert. Der Assay wurde unter Standardbedingungen wie oben beschrieben in der Anwesenheit von 2mM 3-AT durchgeführt. Als Ligand diente der natürliche Agonist Bradykinin (Sigma); Inkubation erfolgte für 20h. Aus Fig. 4 wird ersichtlich, dass der Bradykinin B2 Rezeptor fast gar nicht an den Pheromone Response Pathway koppeln kann, wenn nur Gpa1p zur Verfügung steht. Wenn dagegen die Hefe die Gα-Transplants Gpa1/Gα16 oder Gpa1/Gαq exprimiert, ist eine Anbindung des Rezeptors an diesen Signaltransduktionsweg erfolgreich. Gpa1/Gαq erlaubt die effektivste Kopplung, was zu erwarten war, da der humane Bradykinin B2 Rezeptor in seiner natürlichen Zellumgebung an Gαq koppelt (Hall, Pharmacol.Ther. 56, 131-190 (1992)).

### Beispiel 4:

### Verwendung des Doppel-Reportergen-Assays für Antagonisten-Assays

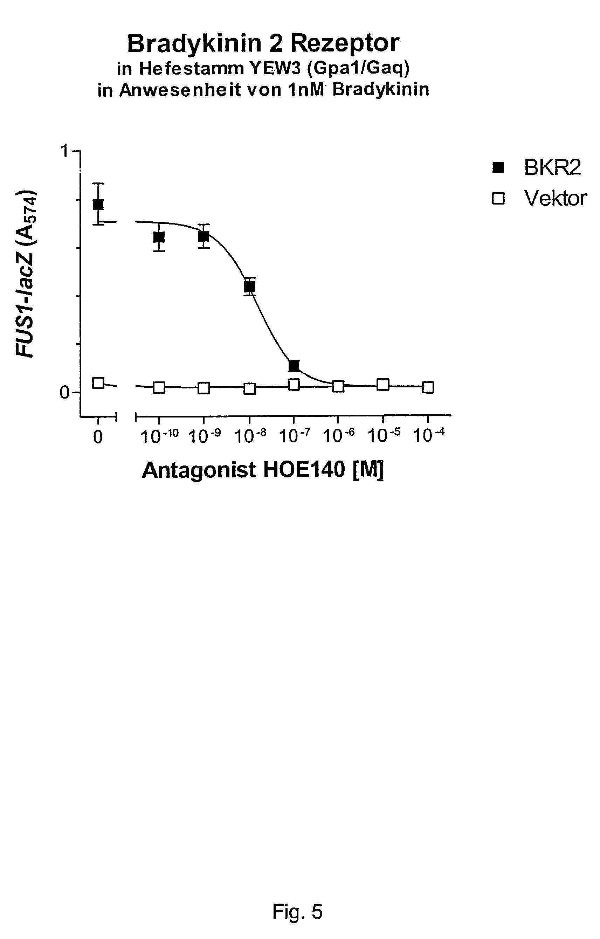

Der Hefestamm YEW3 (Gpa1/Gαq) wurde entweder mit dem leeren Vektor p426GPD oder dem humanen Bradykinin B2 Rezeptor, der in p426GPD kloniert war, transformiert. Der Assay wurde analog zu Beispiel 2 durchgeführt. Der einzige Unterschied war, dass in jeder Vertiefung des Testplatte 1 nM des Agonisten Bradykinin vorhanden war, wozu Verdünnungen des Antagonisten HOE140 (Sigma; Hall, Gen.Pharmacol. 28, 1-6 (1997)) zugegeben wurden. Fig. 5 zeigt, dass steigende Mengen HOE140 das von Bradykinin hervorgerufene Signal auf das Hintergrundniveau herunterdrücken. Der Doppel-Reportergen-Assay eignet sich also auch für Antagonisten-Assays.

### Beispiel 5:

### Vergleich zwischen den Promotoren von FUS1 und YNL279w

Die Hefestämme YEW3 (Gpa1/Gαq, FUS1-Promotor) und YEW17 (Gpa1/Gαq, YNL279w-Promotor) wurden entweder mit dem leeren Vektor p426GPD oder dem humanen Bradykinin B2 Rezeptor, der in p426GPD kloniert war, transformiert. Der Assay wurde unter Standardbedingungen wie oben beschrieben in der Anwesenheit von 2mM 3-AT im Fall von YEW3 und ohne 3-AT im Fall von YEW17 durchgeführt. Nach Zugabe von Assay Mix wurde die Messung der β-Galaktosidase-Aktivität nach 2h und nochmals nach 29h gemessen.

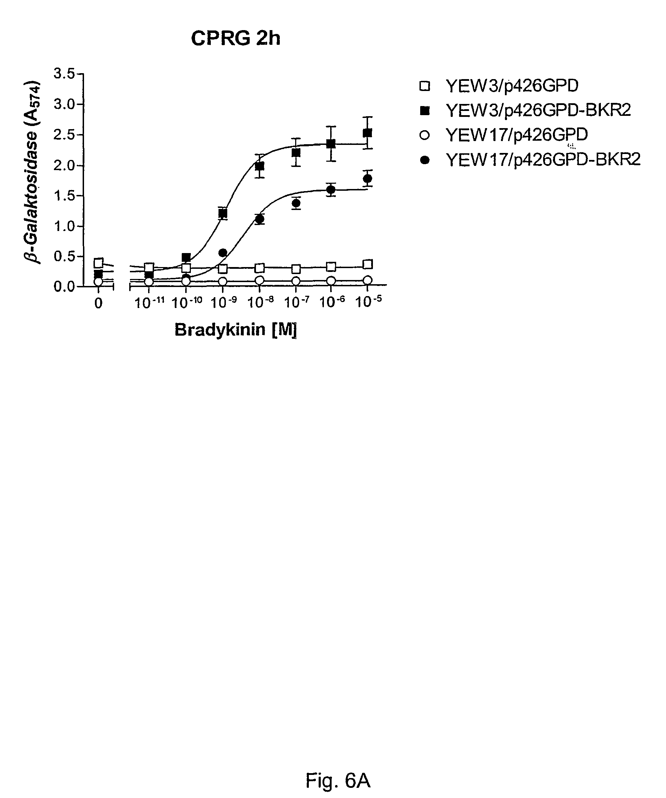

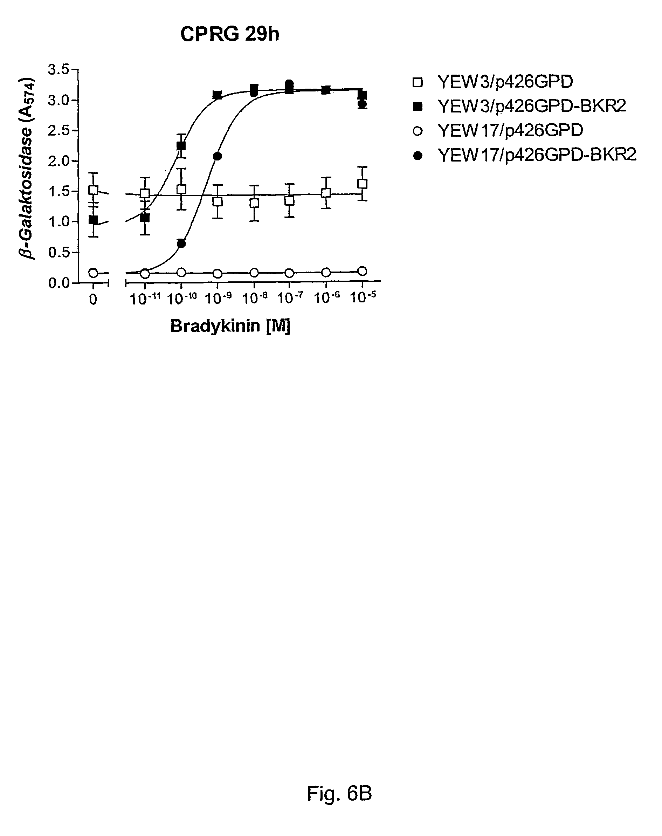

Wie Fig. 6A und noch eindrücklicher Fig. 6B zeigen erhöht sich im Fall des FUS1-Promotors das Hintergrundsignal des mit Rezeptor transformierten Stammes oder auch des mit leerem Kontrollvektor transformierten Stammes über die Zeit erheblich, obwohl sogar 3-AT im Medium vorhanden war. Im Fall des YNL279w-Promotors ändert sich das Hintergrundsignal nicht wesentlich über die Zeit. Die Zugabe von 3-AT ist nicht nötig. Daraus ist abzuleiten, dass die Regulation von YNL279w sehr strikt ist. Dies erweist sich besonders bei High-Throughput-Assays als vorteilhaft, da der Zeitpunkt der Messung nicht präzise eingehalten werden muss, was ein flexibleres Arbeiten ermöglicht.

### Beispiel 6:

### Durchführung eines Assays im Mehrfachrezeptor-Format (Multiplex-Format)

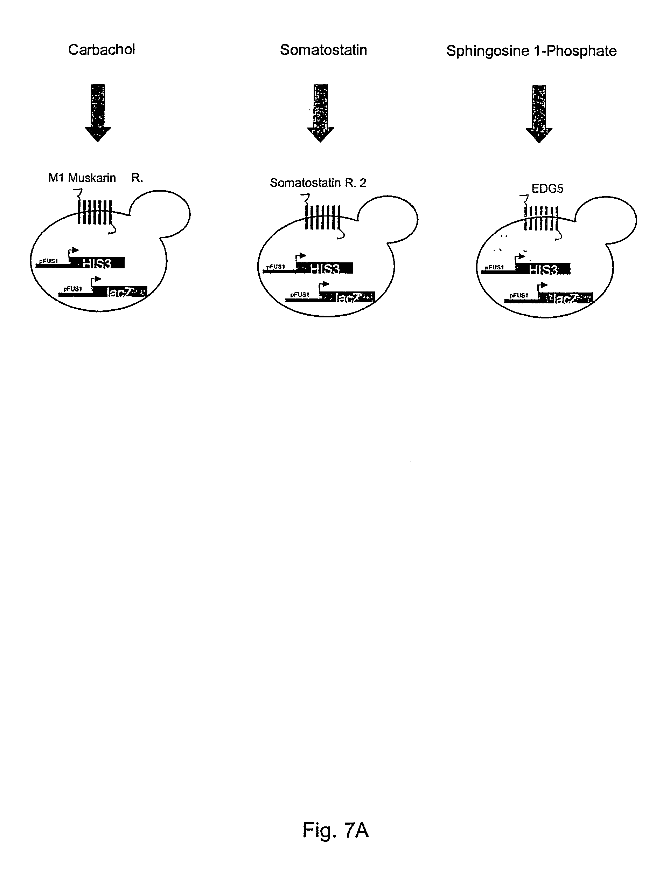

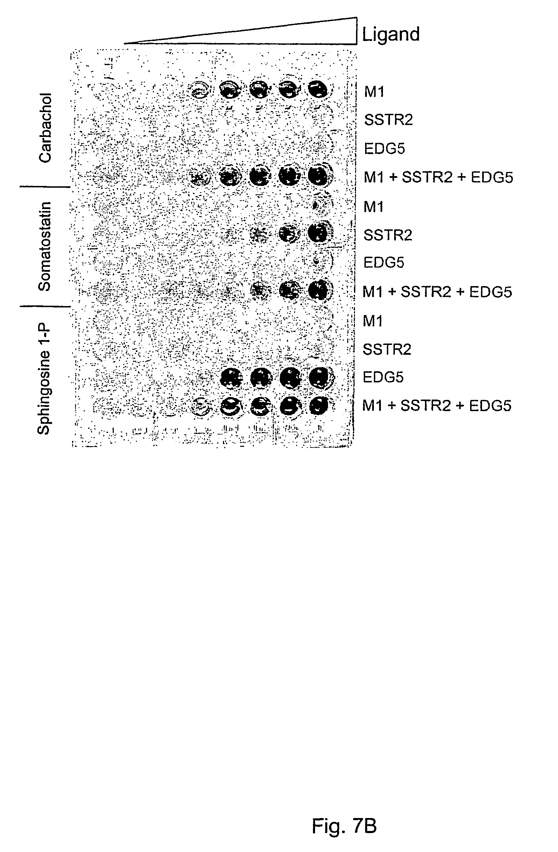

YEW3 (Gpa1/Gαq) und wurde mit dem humanen M1 Muskarinrezeptor und YLJ21 (Gpa1) wurde mit dem Somatostatin Rezeptor 2 und EDG5 transformiert. Die Rezeptoren waren in p426GPD kloniert. Die Durchführung erfolgte nach der oben beschriebenen Standardmethode. Die Rezeptoren wurden entweder einzeln oder im Gemisch getestet. Im Fall des Gemisches wurden die gleichen Übernachtkulturen wie bei den Einzeltests verwendet. Sie wurden erst bei der 1:100 Verdünnung in SC/Gluc-Ura-His pH6.8 gemischt, d.h. das Gemisch enthält somit insgesamt dreimal so viele Zellen wie die Einzeltests. Fig. 7A soll verdeutlichen, dass die Rezeptoren alle individuell, d.h. nicht gemeinsam in einer Zelle exprimiert wurden. Die Inkubation mit den Liganden Carbachol (Sigma; Verdünnung 10⁻⁸M bis10⁻²M), Somatostatin-14 (Bachem; 10⁻¹⁰M bis10⁻⁴M) und Sphingosin-1-phosphat (Biomol, 10⁻¹⁰M bis10⁻⁴M) erfolgte für 24h. Aus Fig. 7B ist ersichtlich, dass eine Detektion der Agonisten auch im Gemisch möglich ist.

### Beispiel 7:

### Vergleich zweier Detektionsmethoden von β-Galaktosidase

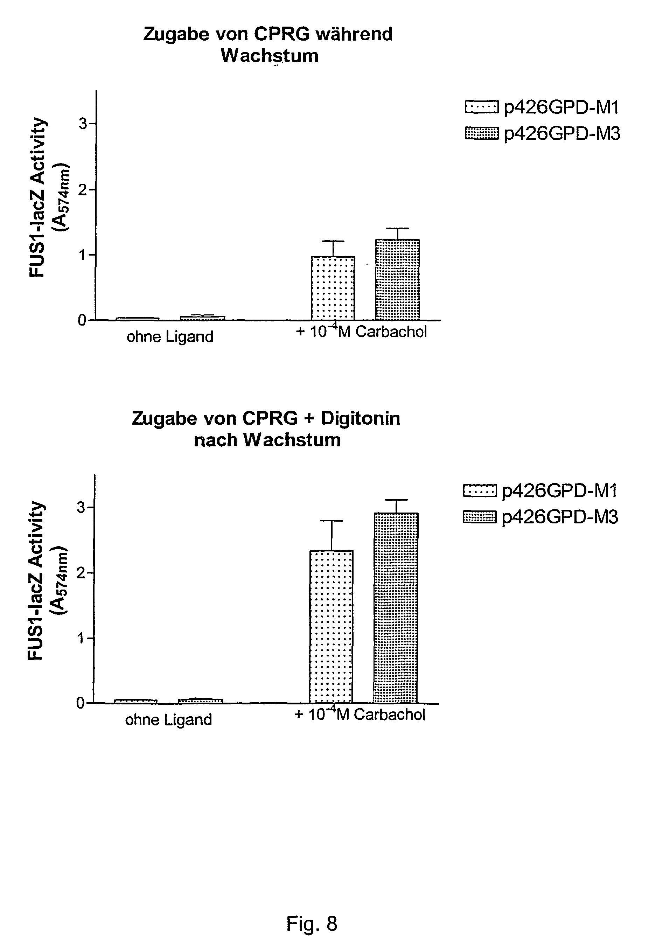

Herkömmlicherweise wird ein Hefe-CPRG-Assay so durchgeführt, dass CPRG während der ganzen Zeit, in der die mit Rezeptor transformierte Hefezelle mit Ligand in Kontakt ist, im Medium vorhanden ist (Brown et al., Yeast 16, 11-22 (2000) und WO99/14344). Fig. 8 zeigt einen Vergleich zwischen dieser Methode und der in dieser Erfindung dargelegten Methode.

YEW3 (Gpa1/Gαq) und wurde mit den humanen M1 und M3 Muskarinrezeptoren, die in p426GPD kloniert waren, transformiert. Übernachtkulturen wurden wie beschrieben angezüchtet und dann in zwei verschiedene Medien 1:100 auf OD₆₀₀0.02 verdünnt. In einem Fall (Fig. 8A) wurden die Zellen in SC/ Gluc-Ura-His, 2mM 3-AT, 0,1mg/ml CPRG, 0,1 M Natriumphosphat-Buffer pH7, verdünnt. Im anderen Fall wurde der Assay wie oben beschrieben in der Anwesenheit vom 2mM 3-AT durchgeführt (Fig. 8B). Als Ligand für die Muskarinrezeptoren wurde Carbachol verwendet. Der Assay wurde für die Daten in Fig. 8A für 28h durchgeführt, bevor die Assayplatte im Photometer gemessen wurde. Im Fall Fig. 8B wurde 26h inkubiert und dann Assay Mix zugegeben. Die Messung erfolgte 2h später.

Wie aus Fig. 8 ersichtlich ist, verbessert die Zugabe von CPRG und einem Detergenz, in diesem Fall Digitonin, erst nach der erfolgten Inkubation mit Ligand, die Leistungsfähigkeit des Assays erheblich. Ein weiterer Vorteil des hier beschriebenen Verfahrens ist die Tatsache, dass eine mögliche Wechselwirkung von CPRG während der langen Inkubation mit chemischen Verbindungen, v.a. während eines Screens, ausgeschlossen ist.

## Patentansprüche

1. Doppel-Reportergen-Assay zur Identifizierung von Substanzen, die fähig sind, die Aktivität eines Zielmoleküls zu beeinflussen, wobei die zu analysierenden Hefezellen zwei Reportergene tragen, und die Aktivität des Zielmoleküls sich auf die Zellvermehrung auswirkt, mit den Schritten
a) Kontaktierung mindestens einer Zelle mit der zu testenden Substanz,
b) Detektion der Zellvermehrung,
c) Detektion der Aktivität desr Reportergenproduktes,
**dadurch gekennzeichnet, dass** ein Reportergen ein Wachstumsmarker und das andere Reportergen ein Enzym ist, dessen Aktivität anhand der Umsetzung eines Substrates detektierbar ist, wobei das Substrat zeitlich versetzt nach Zugabe der zu analysierenden Substanz zugegeben wird.

2. Verfahren nach Anspruch 1, **dadurch gekennzeichnet, dass** sich die Aktivität des Zielmoleküls auf die Aktivität, vorzugsweise die Expression des Reportergenproduktes auswirkt.

3. Verfahren nach Anspruch 1, **dadurch gekennzeichnet, dass** das Zielmolekül ein heterologes Molekül, vorzugsweise ein heterologes Oligonukleotid, Polynukleotid, eine Nukleinsäure, ein Polypeptid, Protein oder Proteinfragment ist.

4. Verfahren nach Anspruch 1, **dadurch gekennzeichnet, dass** das zwischen Zugabe der zu analysierenden Substanz und der des Substrats liegende Zeitintervall der Dauer mindestens eines Zellzyklusdurchlaufs der verwendeten Zelle, vorzugsweise von 2 bis 24 Zellzyklusdurchläufen entspricht.

5. Verfahren nach Anspruch 1, bei dem die Detektion der Aktivität des Reportergenproduktes unter Aufschluss der Zelle erfolgt, vorzugsweise durch Zugabe des Substrates zusammen mit einer die Zellwand permeabel machenden oder zerstörenden Substanz.

6. Verfahren nach einem der vorstehenden Ansprüche, **dadurch gekennzeichnet, dass** das Zielmolekül selbst Einfluss auf die Zellvermehrung ausübt.

7. Verfahren nach einem der vorstehenden Ansprüche, **dadurch gekennzeichnet, dass** das Zielmolekül den Einfluss auf die Zellvermehrung durch Zwischenschaltung eines direkt die Vermehrung beeinflussenden Moleküls ausübt.

8. Verfahren nach Anspruch 7, **dadurch gekennzeichnet, dass** das Zielmolekül seine Wirkung auf die Zellvermehrung durch Wechselwirkung mit einem chimären Molekül ausübt.

9. Verfahren nach einem der vorstehenden Ansprüche, **dadurch gekennzeichnet, dass** die Hefezellen vom Stamm S. cerevisiae sind.

10. Verfahren nach einem der Ansprüche 2 bis 9, **dadurch gekennzeichnet, dass** in einem Ansatz Zellen mit unterschiedlichen Zielmolekülen gleichzeitig gescreent werden.

## Claims

1. A dual reporter gene assay for identifying substances capable of influencing the activity of a target molecule, with the yeast cells to be analyzed carrying two reporter genes and the activity of the target molecule having an effect on cell propagation, which comprises the steps:
a) contacting at least one cell with the substance to be tested,
b) detecting cell propagation,
c) detecting the activity of the reporter gene products,
wherein one reporter gene is a growth marker and the other reporter gene is an enzyme whose activity is detectable on the basis of conversion of a substrate, the substrate being added with a delay after addition of the substance to be analyzed.

2. The method as claimed in claim 1, wherein the activity of the target molecule has an effect on the activity, preferably the expression, of the reporter gene product.

3. The method as claimed in claim 1, wherein the target molecule is a heterologous molecule, preferably a heterologous oligonucleotide, polynucleotide, nucleic acid, polypeptide, protein or protein fragment.

4. The method as claimed in claim 1, wherein the time interval between the addition of the substance to be analyzed and that of the substrate corresponds to the duration of the cell used completing at least one cell cycle, preferably from 2 to 24 cell cycles.

5. The method as claimed in claim 1, wherein the activity of the reporter gene product is detected by way of disrupting the cell, preferably by adding the substrate together with a substance which permeabilizes or destroys the cell wall.

6. The method as claimed in any of the preceding claims, wherein the target molecule itself exerts an influence on cell propagation.

7. The method as claimed in any of the preceding claims, wherein the target molecule exerts the influence on cell propagation by interposition of a molecule which directly influences said propagation.

8. The method as claimed in claim 7, wherein the target molecule exerts its action on cell propagation by interaction with a chimeric molecule.

9. The method as claimed in any of the preceding claims, wherein the yeast cells are from the strain S. cerevisiae.

10. The method as claimed in any of claims 2 to 9, wherein, in one approach, cells are screened simultaneously with different target molecules.

## Revendications

1. Essai de gène rapporteur double pour l'identification de substances aptes à influencer l'activité d'une molécule cible, où les cellules de levure à analyser portent deux gènes rapporteurs et l'activité de la molécule cible agit sur la multiplication des cellules, comprenant les étapes de
a) mise en contact d'au moins une cellule avec la substance à tester,
b) détection de la multiplication cellulaire,
c) détection de l'activité des produits du gène rapporteur,
**caractérisé en ce qu'**un gène rapporteur est un marqueur de croissance et l'autre gène rapporteur est une enzyme, dont l'activité est détectable à l'aide de la transformation d'un substrat, où le substrat est ajouté de manière décalée dans le temps après l'addition de la substance à analyser.

2. Procédé selon la revendication 1, **caractérisé en ce que** l'activité de la molécule cible agit sur l'activité, de préférence l'expression du produit du gène rapporteur.

3. Procédé selon la revendication 1, **caractérisé en ce que** la molécule cible est une molécule hétérologue, de préférence un oligonucléotide, un polynucléotide, un acide nucléique, un polypeptide, une protéine ou un fragment de protéine hétérologue.

4. Procédé selon la revendication 1, **caractérisé en ce que** l'intervalle de temps se trouvant entre l'addition de la substance à analyser et celle du substrat correspond à la durée d'au moins un passage dans le cycle cellulaire de la cellule utilisée, de préférence de 2 à 24 passages dans le cycle cellulaire.

5. Procédé selon la revendication 1, dans lequel la détection de l'activité du produit du gène rapporteur se produit avec digestion de la cellule, de préférence par addition du substrat ensemble avec une substance rendant la membrane cellulaire perméable ou dégradant la membrane cellulaire.

6. Procédé selon l'une quelconque des revendications précédentes, **caractérisé en ce que** la molécule cible exerce elle-même une influence sur la multiplication cellulaire.

7. Procédé selon l'une quelconque des revendications précédentes, **caractérisé en ce que** la molécule cible exerce l'influence sur la multiplication cellulaire en intercalant une molécule influençant directement la multiplication.

8. Procédé selon la revendication 7, **caractérisé en ce que** la molécule cible exerce son effet sur la multiplication cellulaire par interaction avec une molécule chimère.

9. Procédé selon l'une quelconque des revendications précédentes, **caractérisé en ce que** les cellules de levure sont de la souche S. cerevisiae.

10. Procédé selon l'une quelconque des revendications 2 à 9, **caractérisé en ce que** dans une charge, on crible simultanément des cellules avec des molécules cibles différentes.
